# EUROPÄISCHE PATENTSCHRIFT

(11) **EP 1 530 945 B1**
(45) Veröffentlichungstag und Bekanntmachung des Hinweises auf die Patenterteilung: **03.10.2012**
(21) Anmeldenummer: 04019647.9
(22) Anmeldetag: 19.08.2004
(51) Int. Cl.: A61B 3/00, A61B 3/15, G01D 5/14, G01B 7/02

(54) **Gerät zur Durchführung von Untersuchungen am menschlichen Auge**
Device for the execution of examinations of the human eye
Dispositif pour effectuer des examens de l'oeil humain

(30) Priorität: 13.11.2003 DE 20317595 U
(43) Veröffentlichungstag der Anmeldung: 18.05.2005
(73) Patentinhaber: Oculus Optikgeräte GmbH, 35582 Wetzlar-Dutenhofen (DE)
(72) Erfinder: Steinmüller, Andreas, D-35435 Wettenberg (DE)
(74) Vertreter: von den Steinen, Axel

(56) Entgegenhaltungen:
- EP-A- 0 867 145
- WO-A-99/56611
- US-A- 6 100 681
- US-A1- 2003 076 477

## Beschreibung

Die Erfindung betrifft ein Gerät zur Durchführung von Untersuchungen am menschlichen Auge nach dem Oberbegriff des Anspruchs 1.

Gattungsgemäß ist an dem Gerät eine Diagnoseeinheit, beispielsweise ein Keratometer oder Pachymeter, vorgesehen, mit der die Untersuchung am Auge durchgeführt wird. Für die eigentliche Untersuchung muss die Diagnoseeinheit dabei jeweils vor dem zu untersuchenden Auge angeordnet werden. Um zu ermöglichen, dass der behandelnde Arzt wahlweise das linke oder das rechte Auge eines Patienten untersuchen kann, ist bei den gattungsgemäßen Geräten ein Gestell vorgesehen, bei dem das Oberteil relativ zum Unterteil in zumindest einer Richtung verstellbar ist. Diese Richtung (X-Richtung) verläuft dabei so, dass die am Oberteil des Gestells befestigte Diagnoseeinheit wahlweise vor dem linken oder rechten Auge des Patienten angeordnet werden kann.

Ein Gerät des Standes der Technik ist bekanntaus der WO-A-99/56611.

Nachteilig an den bekannten Geräten ist es, dass die Zuordnung, aus der hervorgeht, ob sich das Untersuchungsergebnis auf das linke oder rechte Auge bezieht, vom behandelnden Arzt durch Augenschein festgestellt und von Hand, beispielsweise durch Aufschreiben, dokumentiert werden muss. Diese Art der Feststellung und Dokumentation der Zuordnung der Untersuchungsergebnisse ist zum einen relativ zeitaufwendig und sehr fehlerträchtig. Wird die Zuordnung der Untersuchungsergebnisse zum linken beziehungsweise rechten Auge unrichtig dokumentiert, so kann es zu schweren Behandlungsfehlern kommen, beispielsweise das eine an einem Auge notwendigen Heilbehandlungen ungewollt am anderen, gesunden Auge vorgenommen werden.

Ausgehend von diesem Stand der Technik ist es deshalb Aufgabe der vorliegenden Erfindung ein Gerät zur Durchführung von Untersuchungen am menschlichen Auge vorzuschlagen, mit dem der Handhabungskomfort für den behandelnden Arzt und die Dokumentationssicherheit verbessert wird.

Dies Aufgabe wird durch ein Gerät nach der Lehre des Anspruchs 1 gelöst.

Vorteilhafte Ausführungsformen der Erfindung sind Gegenstand der Unteransprüche.

Die Erfindung beruht auf dem Grundgedanken an dem Gerät ein Sensorsystem vorzusehen, mit dem die Lage des Oberteils in X-Richtung relativ zum Unterteil gemessen werden kann. Bei Durchführung einer Untersuchung an einem Auge des Patienten kann aus dem Messergebnis des Sensorsystems somit abgeleitet werden, ob sich die Untersuchung auf das linke oder auf das rechte Auge des Patienten bezieht. Die Untersuchungsergebnisse können dann in sehr einfacher Weise dem jeweiligen Messergebnis des Sensorsystems zugeordnet und bei Bedarf dokumentiert werden, so dass der Arzt die Zuordnung nicht mehr von Hand, beispielsweise durch Aufschreiben, vornehmen muss.

Erfindungsgemäße Geräte sind Teil der Medizintechnik, so dass besondere Anforderungen hinsichtlich der Hygiene und Desinfizierbarkeit herrschen. Es ist deshalb besonders vorteilhaft, daß das Sensorsystem zur Bestimmung der Lage des Oberteils relativ zum Unterteil berührungslos arbeitet. Denn eine berührungslose Funktion des Sensorsystem ermöglicht es, dass die verschiedenen Bestandteile des Sensorsystems nach außen hin gekapselt werden, beispielsweise durch Anordnung in Gehäusen. Unerwünschte Kanten und Spalten, in denen sich Krankheitskeime absetzen können, werden auf diese Weise vermieden.

Verschiedene Untersuchungen haben gezeigt, dass insbesondere Magnetsensorsysteme besonders geeignet zur Verwendung an erfindungsgemäßen Geräten sind. Diese Magnetsensorsysteme weisen zumindest einen Magnetfeldsensor auf, der mit zumindest einem Geberelement, das ein Magnetfeld erzeugt, zusammen wirkt. Magnetfeldsensor beziehungsweise Geberelement sind jeweils am Oberteil beziehungsweise Unterteil oder umgekehrt befestigt, so das durch eine Relativbewegung zwischen Oberteil und Unterteil der Abstand zwischen Magnetfeldsensor und Geberelement vergrößert wird. Entsprechend der Änderung des Abstandes zwischen Magnetfeldsensor und Geberelement verändert sich auch die Magnetfeldstärke, die vom Geberelement ausgeht und vom Magnetfeldsensor gemessen wird. Die vom Magnetfeldsensor gemessene Magnetfeldstärke kann somit dahingehend ausgewertet werden, dass aus dem Messwert der jeweilige Abstand zwischen Oberteil und Unterteil ableitbar ist.

Besonders zuverlässig arbeiten Magnetfeldsensoren, die in der Art von Hallsensoren ausgebildet sind. Zudem sind derartige Hallsensoren als elektronische Standartbauteile verfügbar, woraus sich ein erheblicher Kostenvorteil ergibt.

In welcher Art das Geberelement ausgebildet ist, ist grundsätzlich beliebig. Nach einer ersten bevorzugten Ausführungsform ist das Geberelement in der Art eines Dauermagneten ausgebildet. Derartige Dauermagneten haben den Vorteil, dass sie zur Erzeugung des notwendigen Magnetfeldes nicht mit Energie versorgt werden müssen, so dass sich der Einbau der Dauermagneten in das erfindungsgemäße Gerät entsprechend vereinfacht.

Alternativ zur Verwendung von Dauermagneten können auch Magnetspulen als Geberelement eingesetzt werden. Das mit solchen Magnetspulen erzeugbare Magnetfeld kann sehr differenziert gestaltet und beeinflusst werden. Außerdem sind Magnetfelder mit größerer Feldstärke in einfacher Weise erzeugbar.

Vorzugsweise sollte der Magnetfeldsensor am Oberteil und das Geberelement am Unterteil des Gestells befestigt sein. Dies insbesondere dann, wenn das Geberelement in der Art eines Dauermagneten ausgebildet ist und somit keine eigene Versorgung mit elektrischer Energie benötigt. Die Versorgung des Magnetfeldsensors mit der erforderlichen elektrischen Energie ist im Oberteil ohne weiteres Möglich, da entsprechende Versorgungsleitungen ohnehin für die Versorgung der üblichen Diagnoseeinheiten durch das Oberteil durchgeführt werden müssen.

Eine besonders einfache Signalauswertung ergibt sich, wenn das Magnetsensorsystem eine linke Hälfte mit einem Magnetfeldsensor und einem Geberelement und eine rechte Hälfte mit ebenfalls einem Magnetfeldsensor und einem Geberelement aufweist. Zur Lagebestimmung des Oberteils relativ zum Unterteil wird dann die Signaldifferenz zwischen der linken Hälfte und der rechten Hälfte des Magnetsensorsystems gemessen. Das Magnetsensorsystem wir dabei vorzugsweise so kalibriert, dass die Signaldifferenz in der Mittelstellung des Oberteils den Wert Null einnimmt. Dies bedeutet mit anderen Worten, dass die Signaldifferenz bei Überschreiten der Mittelstellung das Vorzeichen wechselt und somit aus dem Vorzeichen der gemessenen Signaldifferenz ableitbar ist, ob sich die Diagnoseeinheit zusammen mit dem Oberteil vor dem linken oder vor dem rechten Auge befunden hat.

Um die Reinigung des Gerätes zu vereinfachen sollte das Geberelement beziehungsweise der Magnetfeldsensor innerhalb eines Gehäuses angeordnet sein.

Um die Diagnoseeinheit variabel auf verschiedene Positionen einstellen zu können, ist es besonders vorteilhaft, wenn die Diagnoseeinheit auch horizontal in Y-Richtung und damit in einer Horizontalebene verstellbar ist. Außerdem kann auch noch eine vertikale Verstellung der Diagnoseeinheit in Z-Richtung vorgesehen sein, so dass die Diagnoseeinheit dann innerhalb eines bestimmten Stellbereiches frei im Raum positioniert werden kann.

Das Oberteil kann vorzugsweise aus einem Kreuzschlitten und einem auf dem Kreuzschlitten befestigten Ständer, an dem die Diagnoseeinheit befestigt ist, bestehen. Der Kreuzschlitten selbst ist auf einer entsprechenden Führung am Unterteil in X-Richtung beziehungsweise Y-Richtung verstellbar. Im Ergebnis ermöglicht diese Art der Ausbildung des Oberteils, das unterschiedliche Gerätetypen durch Befestigung verschiedener Diagnoseeinheiten auf dem Kreuzschlitten herstellbar sind, wobei das erfindungsgemäße Sensorsystem jeweils unverändert zur Verfügung gestellt werden kann.

Um die Magnetfeldmessung bei Verwendung eines Magnetsensorsystems nicht zu stören, sollten möglichst wenig Teile des Gerätes aus Eisen hergestellt sein, da das Metall Eisen leicht magnetisierbar ist. Insbesondere sollte der Tisch zur Befestigung des Unterteils des Gerätes aus Nichteisen, insbesondere aus Holz oder Aluminium, hergestellt sein.

Um in einfacher Weise die Zuordnung der verschiedenen Messergebnisse zum jeweils untersuchten linken beziehungsweise rechten Auge einfach dokumentieren zu können, sollte am Gerät eine Speichereinheit, beispielsweise als Bestandteil eines Diagnosecomputers, vorgesehen sein. In dieser Speichereinheit kann das Messergebnis des Sensorsystems beziehungsweise die daraus abgeleiteten Positionsdaten abgespeichert und bei Bedarf wieder abgerufen werden.

In welcher Bauart das Diagnosegerät ausgebildet ist, ist grundsätzlich beliebig. Insbesondere für die Verwendung an Keratometern, Pupillometern, Topografen, Pachymetern, Kataraktanalysatoren und/oder Scheimpflugkameras ist das erfindungsgemäße Sensorsystem geeignet.

Eine Ausführungsform der Erfindung ist in den Zeichnungen schematisch dargestellt und wird nachfolgend beispielhaft erläutert.

Es zeigen:
- **Fig. 1:**: Ein Gerät zur Durchführung von keratografischen Untersuchungen am menschlichen Auge in Ansicht von vorne;
- **Fig. 2:**: Das Gerät gemäß Fig. 1 in seitlicher Ansicht;
- **Fig. 3:**: Das Gerät gemäß Fig. 1 in Ansicht von oben.

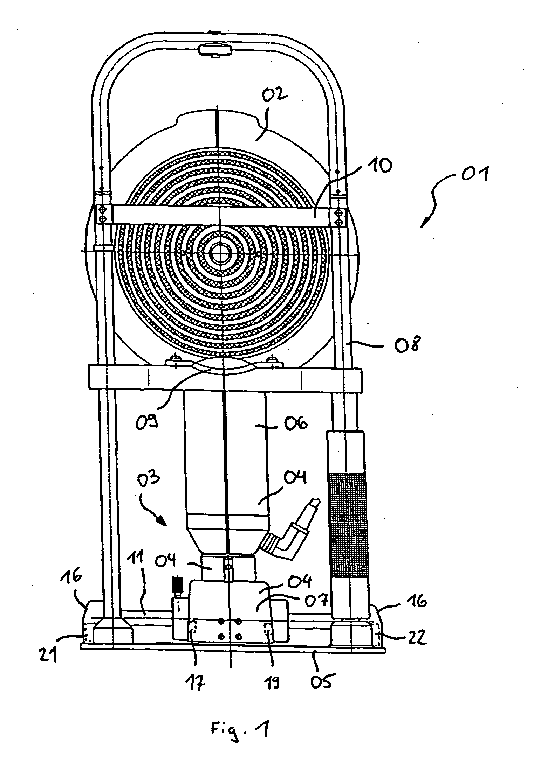

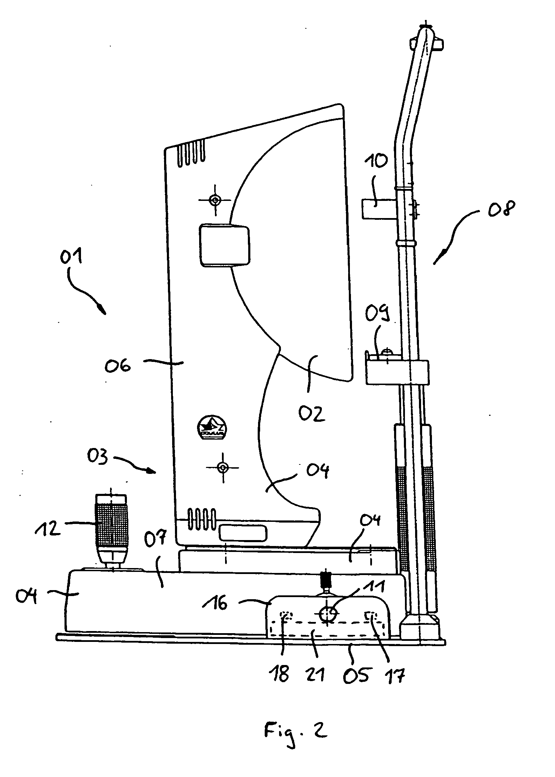

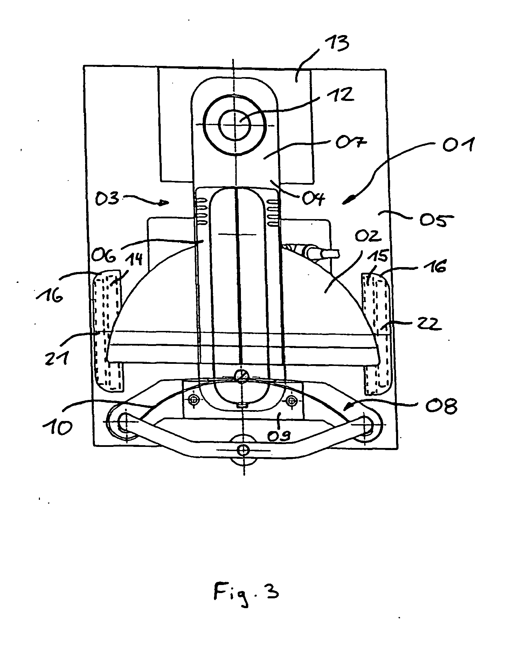

In **Fig. 1** bis **Fig. 3** ist ein Gerät 01 zur Durchführung von keratografischen Untersuchungen am menschlichen Auge, das heißt ein sogenannter Keratograph, in unterschiedlichen Ansichten dargestellt. Als Diagnoseeinheit 02 dient ein Leuchtschirm, mit dem konzentrische Kreise unterschiedlicher Beleuchtungsintensität auf das menschliche Auge projiziert werden können. Mit einer im Mittelpunkt der Diagnoseeinheit 02 platzierten Aufnahmeeinheit, beispielsweise einer CD-Kamera, kann das auf das Auge projizierte Beleuchtungsmuster aufgenommen und die Eigenschaften des Auges entsprechend analysiert werden.

Die Diagnoseeinheit 02 ist an einem Gestell 03 befestigt, das aus einem beweglich gelagerten Oberteil 04 und einem ortsfest angeordneten Unterteil 05 besteht. Das Oberteil 04 seinerseits ist aus einem Ständer 06, der die Diagnoseeinheit 02 trägt, und einem in X-Richtung und Y-Richtung in der Horizontalebene verstellbaren Kreuzschlitten 07 zusammengesetzt. Das Unterteil 05 und der Kreuzschlitten 07 bilden ein Standardmodul, auf dem verschiedene Diagnoseeinheiten, beispielsweise ein Pachymeter oder ein Pupillometer, unter Verwendung entsprechend angepasster Ständer befestigt werden können, um dadurch verschiedenen Gerätetypen zu bilden, an denen jeweils ein erfindungsgemäßes Sensorsystem vorhanden ist.

Vor dem Gerät 01 ist ein Auflager 08 am Unterteil 05 befestigt, das zur Lagefixierung des Kopfes des zu untersuchenden Patienten vor der Diagnoseeinheit 02 dient. Am Auflager 08 ist eine Kinntraverse 09 auf die das Kinn des Patienten aufgelegt werden kann, und eine Stirntraverse 10 an die die Stirn des Patienten angelegt werden kann vorgesehen. Kinntraverse 09 und Stirntraverse 10 können abhängig von der Statur des zu untersuchenden Patienten in der Höhe am Auflager 08 verstellt werden.

Zum Antrieb des Oberteils 04 in Y-Richtung relativ zum Unterteil 05 ist eine Welle 11 vorgesehen, die sich in mit ihrer Längsachse X-Richtung erstreckt. Der Kreuztisch 07 kann auf dieser Welle 11 in X-Richtung verschoben werden, wozu entsprechende Gleitbuchsen im Kreuztisch 07 eingebaut sind. Die für die Verstellung in X-Richtung notwendige Kraft wird vom Behandler über ein Knauf 12 aufgebracht. Unterhalb des Knaufs 12 liegt der Kreuztisch 07 auf einer polierten Stahlplatte 13 auf, so dass das Oberteil 04 nach hinten hin abgestützt ist.

Bei Verstellung des Oberteils 04 in Y-Richtung wird die Welle 11 rotatorisch angetrieben. An den Enden der Welle 11 sind Zahnräder befestigt, die mit am Unterteil 05 befestigten Zahnstangen 14 beziehungsweise 15 in Eingriff stehen. Die Zahnstangen 14 und 15 werden nach außen hin durch Gehäuse 16 abgedeckt.

Zur Lagebestimmung der Lage des Oberteils 04 in X-Richtung relativ zum Unterteil 05 dient an dem Gerät 01 ein Magnetsensorsystem, das aus vier Magnetfeldsensoren 17, 18, 19 und 20 (Magnetfeldsensor 20 in Fig. 1, Fig. 2 und Fig. 3 nicht dargestellt) und zwei den Magnetfeldsensoren 17 bis 20 jeweils paarweise zugeordnete Geberelementen 21 und 22 besteht. Alle Magnetfeldsensoren 17 bis 20 sind in der Art von Hallsensoren ausgebildet und werden von außen unsichtbar auf der Innenseite eines Gehäuses 23 am Oberteil 04 befestigt. Als Geberelemente 21 und 22 kommen stabförmige Dauermagnete zum Einsatz, die im Hohlraum der Gehäuse 16 hinter den Zahnstangen 14 und 15 angeordnet sind.

Das Geberelement 21 ist den beiden Magnetfeldsensoren 17 und 18 zugeordnet und bildet zusammen mit diesen beiden Magnetfeldsensoren die linke Hälfte des Magnetfeldsystems. Das Geberelement 22 ist den beiden Magnetfeldsensoren 19 und 20 zugeordnet und bildet zusammen mit diesen beiden Magnetfeldsensoren die rechte Hälfte des Magnetsensorsystems. Mit den beiden Magnetfeldsensoren 17 und 18 kann der Abstand zwischen dem Oberteil 04 und dem Geberelement 21 gemessen beziehungsweise aus dem Messergebnis abgeleitet werden. Mit den Magnetfeldsensoren 19 und 20 kann der Abstand des Oberteils 04 zum Geberelement 22 gemessen beziehungsweise aus den Messergebnissen abgeleitet werden. Bei Kalibrierung des Magnetsensorsystems werden die verschiedenen Magnetfeldsensoren derart eingestellt, dass die Differenz zwischen dem Sensormesssignal der linken Hälfte und der rechten Hälfte des Magnetsensorsystems gerade in der Mittelstellung des Oberteils 04 den Wert Null einnimmt. Wird nun das Oberteil 04 vor dem linken Auge des Patienten angeordnet, verstärkt sich das Messsignal der linken Hälfte des Magnetfeldsensorsystems wohingegen sich das Messsignal der rechten Hälfte des Magnetfeldsystems verringert. Dementsprechend nimmt die Signaldifferenz ein positives Vorzeichen an. Nach Durchführung der entsprechenden Untersuchung am linken Auge wird dieses Messergebnis des Magnetsensorsystems zusammen mit den Untersuchungsergebnissen abgespeichert, so dass diese Untersuchungsergebnisse eindeutig dem linken Auge des Patienten zugeordnet sind. Bei Untersuchung des rechten Auges ergibt sich entsprechend ein negatives Vorzeichen der Signaldifferenz, so dass aus dem Vorzeichen der Signaldifferenz wiederum die eindeutige Zuordnung der Untersuchungsergebnisse zum rechten Auge des Patienten zuordenbar ist.

## Patentansprüche

1. Gerät (01) zur Durchführung von Untersuchungen am menschlichen Auge, mit einer Diagnoseeinheit (02), die bei Durchführung der Untersuchungen vor dem linken oder rechten Auge angeordnet wird, und mit einem Gestell (03), das ein Oberteil (04) und ein Unterteil (05) aufweist, wobei das Unterteil (05) ortsfest angeordnet wird, und wobei das Oberteil (04) die Diagnoseeinheit (02) trägt und zur Positionierung der Diagnoseeinheit (02) vor dem linken oder rechten Auge relativ zum Unterteil (05) in X-Richtung verstellt werden kann, wobei am Gerät (01) zumindest ein Sensorsystem (17, 18, 19, 20, 21, 22) vorgesehen ist, mit dem die Lage des Oberteils (04) in X-Richtung relativ zum Unterteil (05) gemessen werden kann, wobei das Sensorsystem (17, 18, 19, 20, 21, 22) berührungslos arbeitet,
**dadurch gekennzeichnet,**
**dass** das Sensorsystem in der Art eines Magnetsensorsystems (17, 18, 19, 20, 21, 22) ausgebildet ist, wobei im Magnetsensorsystem zumindest ein Magnetfeldsensor (17, 18, 19, 20) mit zumindest einem Geberelement (21, 22), das ein Magnetfeld erzeugt, zusammenwirkt, wobei das Magnetsensorsystem eine Linke Hälfte (17, 18, 21), in der zumindest ein Magnetfeldsensor (17, 18) mit zumindest einem Geberelement (21) zusammenwirkt, und eine rechte Hälfte (19, 20, 22), in der zumindest ein Magnetfeldsensor (19, 20) mit zumindest einem Geberelement (22) zusammenwirkt, aufweist, wobei zur Lagebestimmung des Oberteils (04) relativ zum Unterteil (05) die Signaldifferenz zwischen linker Hälfte (17, 18, 21) und rechter Hälfte (19, 20, 22) des Magnetsensorsystems gemessen wird.

2. Gerät nach Anspruch 1,
**dadurch gekennzeichnet,**
**dass** der Magnetfeldsensor (17, 18, 19, 20) in der Art eines Hallsensors ausgebildet ist.

3. Gerät nach Anspruch 1 oder 2,
**dadurch gekennzeichnet,**
**dass** das Geberelement (21, 22) in der Art eines Dauermagneten ausgebildet ist.

4. Gerät nach Anspruch 3,
**dadurch gekennzeichnet,**
**dass** der Dauermagnet (21, 22) stabförmig ausgebildet ist.

5. Gerät nach Anspruch 1 oder 2,
**dadurch gekennzeichnet,**
**dass** das Geberelement in der Art einer Magnetspule ausgebildet ist.

6. Gerät nach einem der Ansprüche 1 bis 5,
**dadurch gekennzeichnet,**
**dass** der Magnetfeldsensor (17, 18, 19, 20) am beweglich gelagerten Oberteil (04) und das Geberelement (21, 22) am ortsfest angeordneten Unterteil (05) befestigt ist.

7. Gerät nach Anspruch 1 bis 6,
**dadurch gekennzeichnet,**
**dass** das Geberelement (22) der rechten Hälfte (19, 20, 22) des Magnetsensorsystems (17, 18, 19, 20, 21, 22) und/oder das Geberelement (21) der linken Hälfte (17, 18, 21) des Magnetsensorsystems (17, 18, 19, 20, 21, 22) seitlich links bzw. seitlich rechts des Oberteils (04) in einem Gehäuse (16) am Unterteil (05) angeordnet ist.

8. Gerät nach Anspruch 7,
**dadurch gekennzeichnet,**
**dass** in dem Gehäuse (16) zusammen mit dem Gcberelement (21, 22) jeweils eine Zahnstange (14, 15) angeordnet ist, die zur Verstellung des Oberteils (04) in Y-Richtung mit einem Zahnrad an einer Welle (11) zusammenwirkt.

9. Gerät nach einem der Ansprüche 1 bis 8,
**dadurch gekennzeichnet,**
**dass** der Magnetfeldsensor (17, 18, 19, 20) des Magnetsensorsystems (17, 18, 19, 20, 21, 22) innerhalb eines Gehäuses (23), das Bestandteil des Oberteils (04) ist, angeordnet ist.

10. Gerät nach einem der Ansprüche 1 bis 9,
**dadurch gekennzeichnet,**
**dass** die Diagnoseeinheit (02) horizontal in einer Horizontalebene in Y-Richtung verstellbar ist.

11. Gerät nach Anspruch 10,
**dadurch gekennzeichnet,**
**dass** das Oberteil (04) im wesentlichen aus einem in X-Richtung und Y-Richtung verstellbaren Kreuzschlitten (07) und einem auf dem Kreuzschlitten (07) befestigten Ständer (06), an dem die Diagnosecinhcit (02) befestigt ist, besteht.

12. Gerät nach einem der Ansprüche 1 bis 11,
**dadurch gekennzeichnet,**
**dass** die Diagnoseeinheit vertikal in Z-Richtung verstellbar ist.

13. Gerät nach einem der Ansprüche 1 bis 12,
**dadurch gekennzeichnet,**
**dass** das Unterteil (05) auf einem Tisch aus Nichteisen, insbesondere aus Holz oder Aluminium, montiert ist.

14. Gerät nach einen der Ansprüche 1 bis 13,
**dadurch gekennzeichnet,**
**dass** das Messergebnis des Sensorsystems(17, 18, 19, 20, 21, 22), insbesondere automatisch, in einer Speichereinheit abgespeichert und von dort abgerufen werden kann.

15. Gerät nach einem der Ansprüche 1 bis 14,
**dadurch gekennzeichnet,**
**dass** die Diagnoseeinheit (02) in der Art eines Keratometers und/oder Pupillomctcrs und/oder Topographen und/oder Pachymeters und/oder Kataraktanalysators und/oder einer Scheimpflugkamera ausgebildet ist.

## Claims

1. A device (01) for carrying out examinations of the human eye, comprising a diagnostic unit (02), which, when examinations are carried out, is arranged in front of the left or the right eye, and comprising a frame (03), which has an upper part (04) and a lower part (05), the lower part (05) being arranged in a stationary manner, and the upper part (04) carrying the diagnostic unit (02) and, for positioning of the diagnostic unit (02) in front of the left or the right eye, being adjustable in the X-direction relative to the lower part (05), the device (01) comprising at least one sensor system (17, 18, 19, 20, 21, 22) with which the position of the upper part (04) can be measured in the X-direction relative to the lower part (05), the sensor system (17, 18, 19, 20, 21, 22) operating in a contact-free manner,
**characterised in that**
the sensor system is designed in the manner of a magnet sensor system (17, 18, 19, 20, 21, 22), where, in the magnet sensor system, at least one magnetic field sensor (17, 18, 19, 20) cooperates with at least one pick-up element (21, 22), which generates a magnetic field, the magnetic sensor system having a left half (17, 18, 21), in which at least one magnetic field sensor (17, 18) cooperates with at least one pick-up element (21), and a right half (19, 20, 22), in which at least one magnetic field sensor (19, 20) cooperates with at least one pick-up element (22), the signal difference between the left half (17, 18, 21) and the right half (19, 20, 22) of the magnet sensor system being measured to determine the position of the upper part (04) relative to the lower part (05).

2. The device according to claim 1,
**characterised in that**
the magnetic field sensor (17, 18, 19, 20) is designed in the manner of a Hall-effect sensor.

3. The device according to claim 1 or 2,
**characterised in that**
the pick-up element (21, 22) is designed in the manner of a permanent magnet.

4. The device according to claim 3,
**characterised in that**
the permanent magnet (21, 22) is bar-shaped.

5. The device according to claim 1 or 2,
**characterised in that**
the pick-up element is designed in the manner of a magnet coil.

6. The device according to one of claims 1 to 5,
**characterised in that**
the magnetic field sensor (17, 18, 19, 20) is fastened to the movably mounted upper part (04), and the pick-up element (21, 22) is fastened to the lower part (05), which is arranged in a stationary manner.

7. The device according to claims 1 to 6,
**characterised in that**
the pick-up element (22) of the right half (19, 20, 22) of the magnet sensor system (17, 18, 19, 20, 21, 22) and/or the pick-up element (21) of the left half (17, 18, 21) of the magnet sensor system (17, 18, 19, 20, 21, 22) is/are arranged laterally left or laterally right of the upper part (04) in a housing (16) on the lower part (05).

8. The device according to claim 7,
**characterised in that**,
in each case, a toothed rack (14, 15) is arranged in the housing (16), together with the pick-up element (21, 22), and cooperates with a toothed wheel on a shaft (11) to adjust the upper part (04) in the Y-direction.

9. The device according to one of claims 1 to 8,
**characterised in that**
the magnetic field sensor (17, 18, 19, 20) of the magnet sensor system (17, 18, 19, 20, 21, 22) is arranged within a housing (23), which forms part of the upper part (04).

10. The device according to one of claims 1 to 9,
**characterised in that**
the diagnostic unit (02) is horizontally adjustable in a horizontal plane in the Y-direction.

11. The device according to claim 10,
**characterised in that**
the upper part (04) basically consists of a cross slide (07), which is adjustable in the X-direction and the Y-direction, and of a stand (06), which is fastened on said cross slide (07) and to which the diagnostic unit (02) is fastened.

12. The device according to one of claims 1 to 11,
**characterised in that**
the diagnostic unit is vertically adjustable in the Z-direction.

13. The device according to one of claims 1 to 12,
**characterised in that**
the lower part (05) is mounted on a table made from a non-ferrous material, in particular from wood or aluminium.

14. The device according to one of claims 1 to 13,
**characterised in that**
the measured result of the sensor system (17, 18, 19, 20, 21, 22) can be stored, in particular automatically, in a storage unit and can be retrieved therefrom.

15. The device according to one of claims 1 to 14,
**characterised in that**
the diagnostic unit (02) is designed in the manner of a keratometer and/or pupillometer and/or topographer and/or pachymeter and/or cataract analyser and/or a Scheimpflug camera.

## Revendications

1. Appareil (01) pour effectuer des examens de l'oeil humain, comprenant une unité de diagnostic (02) disposée devant l'oeil gauche ou droit lorsque les examens sont effectués, et un support (03) présentant une partie supérieure (04) et une partie inférieure (05), la partie inférieure (05) étant disposée de manière stationnaire, et la partie supérieure (04) supportant l'unité de diagnostic (02) et pouvant être ajustée dans la direction X par rapport à la partie inférieure (05) pour positionner l'unité de diagnostic (02) devant l'oeil gauche ou droit, dans lequel au moins un système de capteur (17, 18, 19, 20, 21, 22) est prévu sur l'appareil (01) et permet de mesurer la position de la partie supérieure (04) dans la direction X par rapport à la partie inférieure (05), dans lequel le système de capteur (17, 18, 19, 20, 21, 22) fonctionne sans contact,
**caractérisé en ce que**
le système de capteur est réalisé à la manière d'un système de capteur magnétique (17, 18, 19, 20, 21, 22), dans lequel dans le système de capteur magnétique, au moins un capteur de champ magnétique (17, 18, 19, 20) coopère avec au moins un élément transmetteur (21, 22) générant un champ magnétique, dans lequel le système de capteur magnétique présente une moitié gauche (17, 18, 21) dans laquelle au moins un capteur de champ magnétique (17, 18) coopère avec au moins un élément transmetteur (21), et une moitié droite (19, 20, 22) dans laquelle au moins un capteur de champ magnétique (19, 20) coopère avec au moins un élément transmetteur (22), dans lequel, pour déterminer la position de la partie supérieure (04) par rapport à la partie inférieure (05), on mesure la différence de signal entre la moitié gauche (17, 18, 21) et la moitié droite (19, 20, 22) du système de capteur magnétique.

2. Appareil selon la revendication 1, **caractérisé en ce que** le capteur de champ magnétique (17, 18, 19, 20) est réalisé à la manière d'un capteur à effet Hall.

3. Appareil selon la revendication 1 ou 2, **caractérisé en ce que** l'élément transmetteur (21, 22) est réalisé à la manière d'un aimant permanent.

4. Appareil selon la revendication 3, **caractérisé en ce que** l'aimant permanent (21, 22) est réalisé en forme de barre.

5. Appareil selon la revendication 1 ou 2, **caractérisé en ce que** l'élément transmetteur est réalisé à la manière d'une bobine magnétique.

6. Appareil selon l'une quelconque des revendications 1 à 5, **caractérisé en ce que** le capteur de champ magnétique (17, 18, 19, 20) est fixé à la partie supérieure (04) logée de manière mobile et l'élément transmetteur (21, 22) est fixé à la partie inférieure (05) disposée de manière stationnaire.

7. Appareil selon les revendications 1 à 6, **caractérisé en ce que** l'élément transmetteur (22) de la moitié droite (19, 20, 22) du système de capteur magnétique (17, 18, 19, 20, 21, 22) et/ou l'élément transmetteur (21) de la moitié gauche (17, 18, 21) du système de capteur magnétique (17, 18, 19, 20, 21, 22) est disposé latéralement à gauche ou latéralement à droite de la partie supérieure (04) dans un boîtier (16) sur la partie inférieure (05).

8. Appareil selon la revendication 7, **caractérisé en ce que** dans le boîtier (16), en même temps que l'élément transmetteur (21, 22), respectivement une crémaillère (14, 15) est disposée qui coopère avec un engrenage sur un arbre (11) pour ajuster la partie supérieure (04) dans la direction Y.

9. Appareil selon l'une quelconque des revendications 1 à 8, **caractérisé en ce que** le capteur de champ magnétique (17, 18, 19, 20) du système de capteur magnétique (17, 18, 19, 20, 21, 22) est disposé à l'intérieur d'un boîtier (23) faisant partie de la partie supérieure (04).

10. Appareil selon l'une quelconque des revendications 1 à 9, **caractérisé en ce que** l'unité de diagnostic (02) peut être ajustée horizontalement dans un plan horizontal dans la direction Y.

11. Appareil selon la revendication 10, **caractérisé en ce que** la partie supérieure (04) se compose substantiellement d'un chariot en croix (07) ajustable dans la direction X et la direction Y et d'un montant (06) fixé au chariot en croix (07) et auquel est fixée l'unité de diagnostic (02).

12. Appareil selon l'une quelconque des revendications 1 à 11, **caractérisé en ce que** l'unité de diagnostic est ajustable verticalement dans la direction Z.

13. Appareil selon l'une quelconque des revendications 1 à 12, **caractérisé en ce que** la partie inférieure (05) est montée sur une table sans fer, en particulier en bois ou en aluminium.

14. Appareil selon l'une quelconque des revendications 1 à 13, **caractérisé en ce que** le résultat de mesure du système de capteur (17, 18, 19, 20, 21, 22) est sauvegardé, en particulier automatiquement, dans une unité de mémoire et peut être récupéré à partir de celle-ci.

15. Appareil selon l'une quelconque des revendications 1 à 14, **caractérisé en ce que** l'unité de diagnostic (020) est réalisée à la manière d'un kératomètre et/ou d'un pupillomètre et/ou d'un topographe et/ou d'un pachymètre et/ou d'un analyseur de cataracte et/ou d'une caméra de Scheimpflug.
